(19) Europäisches Patentamt
European Patent Office
Office européen des brevets

(11) EP 1 794 294 B1

(12) EUROPÄISCHE PATENTSCHRIFT

(45) Veröffentlichungstag und Bekanntmachung des Hinweises auf die Patenterteilung:
**20.07.2011 Patentblatt 2011/29**

(21) Anmeldenummer: **05789512.0**

(22) Anmeldetag: **26.09.2005**

(51) Int Cl.:
***C12N 9/48*** *(2006.01)* ***C12N 15/57*** *(2006.01)*

(86) Internationale Anmeldenummer:
**PCT/EP2005/054806**

(87) Internationale Veröffentlichungsnummer:
**WO 2006/035008 (06.04.2006 Gazette 2006/14)**

# (54) REKOMBINANTE CARBOXYPEPTIDASE B

RECOMBINANT CARBOXYPEPTIDASE B

CARBOXYPEPTIDASE B RECOMBINANTE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.09.2004 EP 04104696**

(43) Veröffentlichungstag der Anmeldung:
**13.06.2007 Patentblatt 2007/24**

(73) Patentinhaber: **Sanofi-Aventis Deutschland GmbH
65926 Frankfurt am Main (DE)**

(72) Erfinder:
- **VAN DEN HEUVEL, Joop
38304 Wolfenbüttel (DE)**
- **BARTUCH, Jörg
38304 Wolfenbüttel (DE)**
- **CORDES, Arno
38239 Salzgitter (DE)**

(74) Vertreter: **von Kreisler Selting Werner
Deichmannhaus am Dom
Bahnhofsvorplatz 1
50667 Köln (DE)**

(56) Entgegenhaltungen:
**EP-A- 1 538 203 WO-A-97/07769
US-A- 5 672 496**

- **DATABASE UniProt [Online] 1. März 1989 (1989-03-01), "Carboxypeptidase B precursor (EC 3.4.17.2)." XP002351663 gefunden im EBI accession no. UNIPROT:CBPB1_PIG Database accession no. CBPB1_PIG**
- **DATABASE Geneseq [Online] 24. August 1993 (1993-08-24), "Human plasma carboxypeptidase B." XP002337284 gefunden im EBI accession no. GSP:AAR36273 Database accession no. AAR36273**
- **VENTURA SALVADOR ET AL: "Mapping the pro-region of carboxypeptidase B by protein engineering. Cloning, overexpression, and mutagenesis of the porcine proenzyme" JOURNAL OF BIOLOGICAL CHEMISTRY, THE AMERICAN SOCIETY OF BIOLOGICAL CHEMISTS, INC.,, US, Bd. 274, Nr. 28, 9. Juli 1999 (1999-07-09), Seiten 19925-19933, XP002148353 ISSN: 0021-9258**
- **LI S-X ET AL: "Cloning and expression of a new rat procarboxypeptidase B gene in Escherichia coli and purification of recombination carboxypeptidase B" PROTEIN PEPTIDE LETTERS, SCHIPHOL, NL, Bd. 10, Nr. 6, Dezember 2003 (2003-12), Seiten 581-590, XP009029264 ISSN: 0929-8665**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 794 294 B1

Printed by Jouve, 75001 PARIS (FR)

SEQUENCE LISTING

<110> Merck Biosciences

<120> CPB

<130> 052083WO

<160> 11

<170> PatentIn version 3.1

<210> 1
<211> 413
<212> DNA
<213> Artificial Sequence

<220>
<223> CPB-Fragment

<400> 1

```
acgaggaatt ccatatgcac cactctggtg aacacttcga aggtgaaaag gttttcagag     60
ttaacgttga agacgaaaac cacattaaca ttttgcacga attggcttct actactcaaa    120
ttgacttctg gaagccagac tctgttactc aaattaagcc acactctact gttgacttca    180
gagttaaggc tgaagacatt ttgactgttg aagacttctt gaagcaaaac gaattgcaat    240
acgaagtttt gattaacaac ttgagatcag ttttggaagc tcaattcgac tccagagtta    300
gaactactgg tcactcttac gaaaagtaca acaactggga aactattgaa gcatggactc    360
aacaagttac ttctgaaaac ccagacttga tttctagaag cgctattggt acc           413
```

<210> 2
<211> 442
<212> DNA
<213> Artificial Sequence

<220>
<223> CPB-Fragment

<400> 2

```
actttcgaag gtagaactat ttacttgttg aaggttggta agccaggttc taacaagcca     60
gctattttca tggactgtgg tttccacgct agagaatgga tttctccagc tttctgtcaa    120
tggttcgtta gagaagctgt tagaacttac ggtagagaaa ttcacatgac tgaattgttg    180
gacaagttgg acttctacgt tttgccagtt ttgaacattg acggttacat ttacacttgg    240
actaagaaca gaatgtggag aaagactagg tctactaacg ctggttcttc ttgtactggt    300
actgacccaa acagaaactt cgacgctggt tggtgttcta ttggtgcttc aagaaaccca    360
tgtgacgaaa cttactgtgg ttctgctgct gaatctgaaa aggaaactaa ggctttggct    420
gacttcatta gaaacaactt gt                                             442
```

<210> 3
<211> 386
<212> DNA
<213> Artificial Sequence

<220>
<223> CPB-Fragment

<400> 3

cgactattaa ggcttacttg actattcact cttactctca aatgatgttg tacccatact 60
cttacgacta caagttgcca gaaaacaacg ctgaattgaa cgctttggct aaggctactg 120
ttaaggaatt ggcttctttg cacggtacta agtattctta cggtccaggt gctactacta 180
tttacccagc tgctggtggt tctgacgact gggcttacga ccaaggtatt aagtactctt 240
tcactttcga attgagagac aagggtagat acggtttcgt tttgccagaa tctcaaattc 300
aaccaacttg tgaagaaact atgttggcta ttaagtacgt tacttcttac gttttggaac 360
acttgtacta accatggatc cagagc 386

<210> 4
<211> 413
<212> DNA
<213> Sus scrofa

<400> 4

acgaggaatt ccatatgcac cactctggtg aacacttcga aggtgaaaag gttttcagag 60
ttaacgttga agacgaaaac gacatttctg aattgcacga attggcttct actagacaaa 120
ttgacttctg gaagccagac tctgttactc aaattaagcc acactctact gttgacttca 180
gagttaaggc tgaagacatt ttggctgttg aagacttctt ggaacaaaac gaattgcaat 240
acgaagtttt gattaacaac ttgagatcag ttttggaagc tcaattcgac tccagatgta 300
gaactactgg tcactcttac gaaaagtaca acaactggga aactattgaa gcatggactg 360
aacaagttac ttctaagaac ccagacttga tttctagaag cgctattggt acc 413

<210> 5
<211> 442
<212> DNA
<213> Sus scrofa

<400> 5

actttcgacg gtgacaacat ttacttgttg aaggttggta agccaggttc taacaagcca 60
gctattttca tggactgtgg tttccacgct agagaatgga tttctcaagc tttctgtcaa 120
tggttcgtta gagacgctgt tagaacttac ggttacgaag ctcacatgac tgagttcttg 180
gacaacttgg acttctacgt tttgccagtt ttgaacattg acggttacat ttacacttgg 240
actaagaaca gaatgtggag aaagactagg tctactaacg ctggttcttc ttgtactggt 300
actgacccaa acagaaactt caacgctggt tggtgtactg ttggtgcttc tgtgaaccca 360
tgtaacgaaa cttactgtgg ttctgctgct gaatctgaaa aggaaactaa ggctttggct 420
gacttcatta gaaacaactt gt 442

<210> 6
<211> 383
<212> DNA
<213> Sus Scrofa

<400> 6

```
cgactattaa ggcttacttg actattcact cttactctca aatgattttg tacccatact     60
cttacgacta caagttgcca gaaaacgacg ctgaattgaa ctctttggct aagggtgctg    120
ttaaggaatt ggcttctttg tacggtactt cttactctta cggtccaggt tctactacta    180
tttacccagc tgctggtggt tctgacgact gggcttacaa ccaaggtatt aagtactctt    240
tcactttcga attgagagac aagggtagat tcggtttcgt tttgccagaa tctcaaattc    300
aagctacttg tcaagaaact atgttggctg ttaagtacgt tactaactac actttggaac    360
acttgtaacc atggatccag agc                                            383
```

<210> 7
<211> 402
<212> PRT
<213> Artificial Sequence

<220>
<223> new CPB

<400> 7

```
His His Ser Gly Glu His Phe Glu Gly Glu Lys Val Phe Arg Val Asn
1               5                   10                  15
Val Glu Asp Glu Asn His Ile Asn Ile Leu His Glu Leu Ala Ser Thr
            20                  25                  30
Thr Gln Ile Asp Phe Trp Lys Pro Asp Ser Val Thr Gln Ile Lys Pro
        35                  40                  45
```

His Ser Thr Val Asp Phe Arg Val Lys Ala Glu Asp Ile Leu Thr Val
    50                  55                  60
Glu Asp Phe Leu Lys Gln Asn Glu Leu Gln Tyr Glu Val Leu Ile Asn
65                  70                  75                  80
Asn Leu Arg Ser Val Leu Glu Ala Gln Phe Asp Ser Arg Val Arg Thr
                85                  90                  95
Thr Gly His Ser Tyr Glu Lys Tyr Asn Asn Trp Glu Thr Ile Glu Ala
            100                 105                 110
Trp Thr Gln Gln Val Thr Ser Glu Asn Pro Asp Leu Ile Ser Arg Ser
        115                 120                 125
Ala Ile Gly Thr Thr Phe Glu Gly Arg Thr Ile Tyr Leu Leu Lys Val
    130                 135                 140
Gly Lys Pro Gly Ser Asn Lys Pro Ala Ile Phe Met Asp Cys Gly Phe
145                 150                 155                 160
His Ala Arg Glu Trp Ile Ser Pro Ala Phe Cys Gln Trp Phe Val Arg
                165                 170                 175
Glu Ala Val Arg Thr Tyr Gly Arg Glu Ile His Met Thr Glu Leu Leu
            180                 185                 190
Asp Lys Leu Asp Phe Tyr Val Leu Pro Val Leu Asn Ile Asp Gly Tyr
        195                 200                 205
Ile Tyr Thr Trp Thr Lys Asn Arg Met Trp Arg Lys Thr Arg Ser Thr
    210                 215                 220
Asn Ala Gly Ser Ser Cys Thr Gly Thr Asp Pro Asn Arg Asn Phe Asp
225                 230                 235                 240
Ala Gly Trp Cys Ser Ile Gly Ala Ser Arg Asn Pro Cys Asp Glu Thr
                245                 250                 255
Tyr Cys Gly Ser Ala Ala Glu Ser Glu Lys Glu Thr Lys Ala Leu Ala
            260                 265                 270
Asp Phe Ile Arg Asn Asn Leu Ser Thr Ile Lys Ala Tyr Leu Thr Ile
        275                 280                 285
His Ser Tyr Ser Gln Met Met Leu Tyr Pro Tyr Ser Tyr Asp Tyr Lys
    290                 295                 300
Leu Pro Glu Asn Asn Ala Glu Leu Asn Ala Leu Ala Lys Ala Thr Val
305                 310                 315                 320
Lys Glu Leu Ala Ser Leu His Gly Thr Lys Tyr Ser Tyr Gly Pro Gly
                325                 330                 335
Ala Thr Thr Ile Tyr Pro Ala Ala Gly Gly Ser Asp Asp Trp Ala Tyr
            340                 345                 350

Asp Gln Gly Ile Lys Tyr Ser Phe Thr Phe Glu Leu Arg Asp Lys Gly
355 360 365

Arg Tyr Gly Phe Val Leu Pro Glu Ser Gln Ile Gln Pro Thr Cys Glu
370 375 380

Glu Thr Met Leu Ala Ile Lys Tyr Val Thr Ser Tyr Val Leu Glu His
385 390 395 400

Leu Tyr

<210> 8
<211> 401
<212> PRT
<213> Sus scrofa

<400> 8

His His Ser Gly Glu His Phe Glu Gly Glu Lys Val Phe Arg Val Asn
1 5 10 15

Val Glu Asp Glu Asn Asp Ile Ser Glu Leu His Glu Leu Ala Ser Thr
20 25 30

Arg Gln Ile Asp Phe Trp Lys Pro Asp Ser Val Thr Gln Ile Lys Pro
35 40 45

His Ser Thr Val Asp Phe Arg Val Lys Ala Glu Asp Ile Leu Ala Val
50 55 60

Glu Asp Phe Leu Glu Gln Asn Glu Leu Gln Tyr Glu Val Leu Ile Asn
65 70 75 80

Asn Leu Arg Ser Val Leu Glu Ala Gln Phe Asp Ser Arg Cys Arg Thr
85 90 95

Thr Gly His Ser Tyr Glu Lys Tyr Asn Asn Trp Glu Thr Ile Glu Ala
100 105 110

Trp Thr Glu Gln Val Thr Ser Lys Asn Pro Asp Leu Ile Ser Arg Ser
115 120 125

Ala Ile Gly Thr Thr Phe Asp Gly Asp Asn Ile Tyr Leu Leu Lys Val
130 135 140

Gly Lys Pro Gly Ser Asn Lys Pro Ala Ile Phe Met Asp Cys Gly Phe
145 150 155 160

His Ala Arg Glu Trp Ile Ser Gln Ala Phe Cys Gln Trp Phe Val Arg
165 170 175

Asp Ala Val Arg Thr Tyr Gly Tyr Glu Ala His Met Thr Glu Phe Leu

```
            180                 185                 190
Asp Asn Leu Asp Phe Tyr Val Leu Pro Val Leu Asn Ile Asp Gly Tyr
        195                 200                 205
Ile Tyr Thr Trp Thr Lys Asn Arg Met Trp Arg Lys Thr Arg Ser Thr
    210                 215                 220
Asn Ala Gly Ser Ser Cys Thr Gly Thr Asp Pro Asn Arg Asn Phe Asn
225                 230                 235                 240
Ala Gly Trp Cys Thr Val Gly Ala Ser Val Asn Pro Cys Asn Glu Thr
                245                 250                 255
Tyr Cys Gly Ser Ala Ala Glu Ser Glu Lys Glu Thr Lys Ala Leu Ala
            260                 265                 270
Asp Phe Ile Arg Asn Asn Leu Ser Thr Ile Lys Ala Tyr Leu Thr Ile
        275                 280                 285
His Ser Tyr Ser Gln Met Ile Leu Tyr Pro Tyr Ser Tyr Asp Tyr Lys
    290                 295                 300
Leu Pro Glu Asn Asp Ala Glu Leu Asn Ser Leu Ala Lys Gly Ala Val
305                 310                 315                 320
Lys Glu Leu Ala Ser Leu Tyr Gly Thr Ser Tyr Ser Tyr Gly Pro Gly
                325                 330                 335
Ser Thr Thr Ile Tyr Pro Ala Ala Gly Gly Ser Asp Asp Trp Ala Tyr
            340                 345                 350
Asn Gln Gly Ile Lys Tyr Ser Phe Thr Phe Glu Leu Arg Asp Lys Gly
        355                 360                 365
Arg Phe Gly Phe Val Leu Pro Glu Ser Gln Ile Gln Ala Thr Cys Gln
    370                 375                 380
Glu Thr Met Leu Ala Val Lys Tyr Val Thr Asn Tyr Thr Leu Glu His
385                 390                 395                 400
Leu
```

<210> 9
<211> 441
<212> DNA
<213> Artificial Sequence

<220>
<223> CPB-Fragment

<400> 9

```
atgttggcgt tcttgattct tgtgactgtg actctagcat ctgctcatca ttctggtgag     60
cactttgaag gtgagaaggt gttccgtgtc aatgttgaag atgaaaatga catcagctta    120
ctccatgagt tggccagcac caggcagatt gacttctgga aaccagattc tgtcacacaa    180
atcaaacctc acagtacagt tgacttccgc gtgaaagcag aagatatttt ggctgtggaa    240
gactttctgg agcagaatga actacaatat gaggtactca taaacaacct gagatctgtg    300
ctagaggctc agtttgacag cagagtccgt acaactggac acagttatga gaagtacaac    360
aactgggaaa cgatagaggc ttggactaag caagtcacca gtgaaaatcc agacctcatc    420
tctcgcacag ccatcggaac t                                              441
```

<210> 10
<211> 442
<212> DNA
<213> Artificial Sequence

<220>
<223> CPB-Fragment

<400> 10

```
acatttttag gaaacaatat atacctcctc aaggttggca aacctggacc aaataagcct     60
gccattttca tggactgtgg tttccatgcc agagaatgga tttcccatgc attttgccag    120
tggtttgtga gagaggctgt tctcacctat ggatatgaga gtcacatgac agaattcctc    180
aacaagctag acttttatgt cttgcctgtg ctcaatattg atggctacat ctacacctgg    240
accaagaacc gaatgtggag aaagacccgc tctaccaatg ctggaactac ctgcattggc    300
acagacccca acagaaattt tgatgctggg tggtgcacaa ctggagcctc tacagacccc    360
tgcgatgaga cttactgtgg atctgctgca gagtctgaaa aagagaccaa ggccctggct    420
gattttatac gcaacaacct ct                                             442
```

<210> 11
<211> 368
<212> DNA
<213> Artificial Sequence

<220>
<223> CPB-Fragment

<400> 11

```
cctccatcaa agcatacctg acgatccact catactcaca gatgatactc tacccttatt     60
cctatgatta caaactcccc gagaacaatg ctgagttgaa taacctggct aaggctgccg    120
tgaaagaact tgctacactg tatggcacca agtacacata cggcccagga gctacaacaa    180
```

```
tctatcctgc tgctgggggc tctgatgact gggcttatga ccaaggaatc aaatattcct    240
tcacctttga actccgggat aaaggcagat atggttttat cctccctgaa tcccagatcc    300
aggcaacctg tgaggaaaca atgctggcca tcaaatacgt aaccaactac gtgctgggcc    360
acctgtaa                                                             368
```

## Beschreibung

Die vorliegende Erfindung betrifft Pro-Carboxypeptidase B und Carboxypeptidase B sowie Verfahren zur ihrer Herstellung.

Carboxypeptidase B (CPB) ist eine pankreatische Exopeptidase, die durch Hydrolyse von Peptidbindungen an basischen Aminosäuren wie Lysin, Arginin und Ornitin spaltet. Die Spaltung erfolgt dabei am C-terminalen Ende der Polypeptide. Es handelt sich um eine zinkenthaltende Peptidase (EC 3.4.17.2).

Carboxypeptidase B entsteht aus einer Preprocarboxypeptidase B, die enzymatisch inaktiv ist. Von der Preprocarboxypeptidase B wird ein Signalpeptid abgespalten, um eine Procarboxypeptidase B zu erhalten, die ebenfalls enzymatisch inaktiv ist. Hiervon wird dann ein weiteres Peptid abgespalten, um zur aktiven Carboxypeptidase zu gelangen.

Das molekulare Gewicht der Carboxypeptidase B beträgt ca. 35 kD. Sie wird für eine Vielzahl von Zwecken eingesetzt, insbesondere der Herstellung von Peptiden wie beispielsweise Insulin und in der Proteinsequenzanalyse. Carboxypeptidase B wird üblicherweise aus Schweinepankreas aufgereinigt.

Die cDNA-Sequenzen der humanen Carboxypeptidase B sind bekannt.

Die WO 96/23064 beschreibt ein Verfahren zur Herstellung von rekombinanter Rattencarboxypeptidase B. Versuche, das dort beschriebene Plasmid zu exprimieren, haben nicht zum gewünschten Erfolg geführt.

Kommerziell erhältliche Carboxypeptidase (gereinigt aus natürlichen Quellen) hat typischerweise Aktivitäten von ca. 50 bis 170 U/mg. 1 U entspricht einer Hydrolyse von 1 mmol Hyppuryl-L-Arg/min bei 25°C und einem pH-Wert von 7,65.

Aus natürlichen Quellen aufgereinigte Carboxypeptidase B weist immer eine Verunreinigung mit geringen Mengen anderer Proteasen auf. Es besteht daher weiterhin ein Bedürfnis nach hochreinen Carboxypeptidasen mit einer möglichst hohen spezifischen Aktivität.

Gelöst wird die Aufgabe durch die Bereitstellung einer neuen Procarboxypeptidase B (Pro-CPB) und einer neuen Carboxypeptidase B (CPB) gemäß den Patentansprüchen. Die erfindungsgemäßen Carboxypeptidasen haben eine Enzymaktivität von mindestens 200 U pro mg, bevorzugt mehr als 250 U pro mg, noch mehr bevorzugt mehr als 270 U pro mg.

Die erfindungsgemäßen Carboxypeptidasen B lassen sich besser aufreinigen. Aus Schweinepankreas gewonnene Carboxypeptidase B hat in der reversed-phase HPLC eine Reinheit von 81,6%, während die erfindungsgemäße CPB eine Reinheit von 97,4% aufweist. In der Gelpermeationschromatographie zeigen die erfindungsgemäßen Carboxypeptidasen eine Reinheit von 99,1% gegenüber einer aus Schweinepankreas aufgereinigte Carboxypeptidase mit einer Reinheit von 77,2%. Durch die geänderte Struktur wird überraschenderweise eine höhere Temperaturstabilität bei 40°C erzielt. Außerdem besteht eine höhere Langzeitstabilität bei einer Lagerung in flüssiger Form bei pH 8.

Gegenstand der Erfindung ist daher zum einen eine Nukleinsäure, codierend für Pro-Carboxypeptidase B (Pro-CPB) umfassend drei Abschnitte A, B und C, wobei der Abschnitt A die Sequenz gemäß Seq. ID. Nr. 1, der Abschnitt B die Sequenz der Seq. ID Nr. 2 und der Abschnitt C die Sequenz gemäß Seq. ID Nr. 3 aufweist.

Besonders bevorzugte Sequenzen für die Nukleinsäure, die für Procarboxypeptidase B codiert, sind

- Seq. ID Nr. 1 - Seq. ID Nr. 2 - Seq. ID Nr. 3

Gegenstand der Erfindung ist weiterhin die Procarboxypeptidase erhältlich durch Expression einer erfindungsgemäßen Nukleinsäure sowie eine Carboxypeptidase B erhältlich durch Abspaltung der pro-Sequenz von erfindungsgernäßen Procarboxypeptidase B. Eine solche Abspaltung ist beispielsweise mit Trypsin möglich.

Ein weiterer Gegenstand der Erfindung ist ein Expressionsvektor enthaltend die erfindungsgemäße Nukleinsäure sowie ein transformierter Organismus enthaltend den erfindungsgemäßen Expressionsvektor.

Ein weiterer Gegenstand der Erfindung ist ein Protein enthaltend ein Aminosäuresequenz gemäß Seq. ID Nr. 8 mit mindestens 5 Mutationen ausgewählt aus der Gruppe D22H, S24N, E25I, R33T, A63T, E69K, C94V, E115Q, K120E, D135E, D137R, N138T, Q168P, D177E, Y184R, A186I, F191L, N194K, N240D, T245S, V246I, V250R, N254D, I295M, D309N, S314A, G318A, A319T, Y327H, S330K, S337A, N353D, F370Y, A381P, Q384E, V390I, N395S, T397V.

In einer Ausführungsform ist als Aminosäure 402 ein Y angehängt.

In einer bevorzugten Ausführungsform weist das erfindungsgemäße Protein mindestens sieben, mehr bevorzugt mindestens zehn und am meisten bevorzugt mindestens fünfzehn der oben genannten Mutationen auf.

Das erfindungsgemäße Protein ist als rekombinantes Protein frei von Verunreinigung durch andere natürliche Proteasen. Darüber hinaus kann es in besonders hoher Reinheit produziert werden, insbesondere Reinheiten größer 170 U pro mg, bevorzugt mehr als 200 U pro mg, noch mehr bevorzugt mehr als 250 U pro mg und am meisten bevorzugt mehr als 280 pro mg.

Ein weiterer Gegenstand der Erfindung ist ein Verfahren zur Expression von Pro-CPB umfassend die Schritte:

- Fermentieren eines transformierten Organismus,
- Induzieren der Expression,
- Aufreinigen der Pro-CPB

sowie ein Verfahren zur Expression von Carboxypeptidase B umfassend die Schritte:

- Fermentieren eines transformierten Organismus gemäß Anspruch 10,
- Induzieren der Expression,
- Aktivierung durch Spaltung der Pro-CPB zu CPB,
- Aufreinigung der CPB.

Ein weiterer Gegenstand der Erfindung ist eine Carboxypeptidase mit der Sequenz gemäß der Seq. ID Nr. 7 .
Mutation bedeutet ein Austausch einer Aminosäure gegen eine andere, eine Insertion die zusätzlich Einfügung einer weiteren Aminosäure und eine Deletion das Entfernen einer Aminosäure.
Ein besonders bevorzugtes Expressionssystem ist *Pichia* pastoris. Grundsätzlich können jedoch auch andere übliche Expressionssysteme wie das Baculovirussystem in Insektenzellen oder eine Expression in Säugetierzellen eingesetzt werden. Der Einsatz des Pichia Expressionssystems ist beispielsweise in US 5,102,789 beschrieben, auf die hier Bezug genommen wird.
Die erfindungsgemäßen Nukleinsäuren können beispielsweise durch chemische Synthese in Fragmenten synthetisiert werden und die Fragmente anschließend ligiert werden. Die erfindungsgemäßen Proteine können dann durch Expression der entsprechenden Nukleinsäure enthalten werden. Die Nukleinsäure kann auch durch Site Directed Mutatgenesis, ausgehend von der bekannten CDNA-Sequenz der CBP, erhalten werden. Wege hierfür sind beispielsweise im *The* Journal of Biological Chemistry, 174 (1999), 19925 - 19933 beschrieben, auf das hier Bezug genommen wird.
Die Erfindung wird durch die weiteren nachfolgenden Beispiele näher erläutert.
Die Gene wurden in folgende Vektoren kloniert:

| | |
|---|---|
| *Pichia* pastoris: | pKINTEX, pKEXTEX, pPiczα |
| *E. coli:* | Tuner(DE3)pET22-OMPA |
| *Arxula adeninovirans:* | pAL-ALEU2m-GAA 1. |

Die höchsten Expressionsraten wurden in *Pichia pastoris* pKEXTEX-npproCPB erzielt.

*Fermentationsverfahren*

Es wurde ein Fed-batch-Verfahren sowie ein kontinuierliches Verfahren entwickelt. Dabei wurde ca. 200 mg/l npproCPB in das Medium sekretiert.

*Fed-batch Fermentation*

Fermentationsmedium *(für 1 L):*

**Hexaphosphatmedium**

25 g Sodiumhexameta Phosphat
9 g Ammoniumsulfat

**Glycerin Salz Medium**

45,6 g Glycerin (86%ig)

18,2 g Kaliumsulfat

14,9 g Magnesiumsulfat-7-Hydrat

0,9 g Calciumsulfat-Dihydrat

PTM1 (Spurenelemente) 1 ml/L

| | | |
|---|---|---|
| **Glycerinfeed (1L)** | | |
| 314 g (86%ig) Glycerin | auf 1000 mL aqua dest | autoklavieren |
| | | Nach Abkühlen Zugabe von 9mL sterilen PTM1 |
| **Methanolfeed (1L)** | | Zugabe von 12mL sterilen PTM1 |
| 1L Methanol | | |

Fermentationsbedingungen

| | | |
|---|---|---|
| Temperatur | 28°C | |
| Rührdrehzahl | 500 bis 1000 U/min | |
| Fermentationszeit | 90,1 bis 138,6 h | |
| Begasung | 0,8 bis 2 vvm | Luft |
| Startvolumen Kulturlösung | 2 bis 8L | Medium und Animpfkultur |
| Animpfvolumen | 10% des Gesamtstartvolumens | Schüttelkultur |
| Sauerstoffpartialdruck | 6 bis 100% | |
| pH-Wert | 4,4 bis 7,3 | |

Fermentationsablauf

| | | |
|---|---|---|
| Glycerinzugabe | Start: | bei Optischer Dichte $OD_{600}$ der Kulturlösung (Extinktion 600nm) zwischen 15 und 140 |
| | Zugaberate: | zwischen 0,4 und 1,8 mL/min Glycerinfeed |
| | Zugabemenge: | zwischen 4,2 und 16,6% im Verhältnis zum Startvolumen |
| Methanolzugabe | Start: | bei $OD_{600}$ zwischen 50 und 195 |
| | Zugaberate: | zwischen 0,04 und 0,2mL/min bei Methanolsteuerung zwischen 0,1 und 3% Methanolgehalt in der Kulturlösung |
| Fermentationsabbruch | $OD_{600}$: | zwischen 144,2 und 510 |

*Kontinuierliche Fermentation*

*Medienbestandteile des kontinuierlichen Feeds (1 L)*

| | | |
|---|---|---|
| 9,8 mL Phosphorsäure (75%) | | |
| 0,2 g Calciumchlorid-Dihydrat | | |
| 6 g Kaliumsulfat | | |

(fortgesetzt)

| | | |
|---|---|---|
| 2,28 g Magnesiumsulfat-7-Hydrat | | |
| 1,35 g Kaliumhydroxid | in 500 mL aqua dest. | |
| 1 mL Struktol SB2122 | | autoklavieren |
| | | |
| 5,4 mg Biotin in Lösung | | sterilfiltriert |
| | | |
| 2,7 mL PTM1 | | sterilfiltriert |
| | | |
| 6 mL Ammoniak (25%) | | |
| | | |
| 239 mL Methanol | | |
| | auf 1000 mL mit autokavierten aqua dest. | |

*Fermentationsablauf*

| | | |
|---|---|---|
| Glycerinzugabe | Start: | bei $OD_{600}$ 16,5 |
| | Zugaberate: | zwischen 1,4 mL/min Glycerinfeed |
| | Zugabemenge: | 21,8% im Verhältnis zum Startvolumen |
| Methanolzugabe | Start: | bei $OD_{600}$ 126,8 |
| | Zugaberate: | 0,23 mL/min |
| | Zugabemenge: | 9% im Verhältnis zum Starvolumen |
| Kontinuierlicher Feed | Start: | bei $OD_{600}$ 130,1 |
| | Zugaberate: | zwischen 20 und 200 mL/h |

*Aufarbeitungsverfahren*

1. Schritt: Aktivierung der npproCPB durch Trypsinspaltung

2. Schritt: Anionenaustauschchromatographie -DEAE-Sephacel

3. Schritt: Hydrophobe Chromatographie -Butyl-Sepharose

Aus diesem Verfahren resultiert eine reine npCPB.

Aktivierung von pronpCPB mittels Trypsinspaltung

| | |
|---|---|
| Trypsin aus | Schweinepankreas 1645 U/mg oder<br>Schweinepankreas 15000 U/mg oder<br>Rinderpankreas 9280 U/mg |

(fortgesetzt)

| | |
|---|---|
| Konzentrationsverhältnisse (Trypsin:pronpCPB) | zwischen 1:1 und 1:1000 |
| pH-Werte | zwischen pH 6,5 und pH 8,5 |
| Spaltungsdauer | Zwischen 10 min und 17 h |
| Temperatur | Zwischen 4 und 30°C |
| Aktivierungszeitpunkt im Aufarbeitungsprozess | - unbehandelter Fermentationsüberstand<br>- nach PEG-Fällung und Dialyse<br>- nach DEAE-Chromatographie |

*Anionenaustauschchromatographie*

| | |
|---|---|
| Anionaustauschergel | DEAE-Sephacel oder Q-Sepharose |
| Säulenvolumen | 5 bis 500mL |
| Elutionspuffer | 20mM Tris/Acetat + 0,1 mM ZnCl2 pH7,S oder pH8 |
| kontinuierlichen Gradient | 0 bis 2S0mM NaCl oder 0 bis 500mM NaCl |
| Stufengradient | zwischen 500 mM und 1000 mM NaCl |
| Gradientenlänge | zwischen 1 und 5fachen Säulenvolumen |
| Beladung (CPB/mL Anionaustauschergel) | zwischen 10 und 64 U/mL |

*Hydrophobe Chromatographie (HIC)*

| | |
|---|---|
| HIC-Gel | Toyopearl Butyl 650M |
| Säulenvolumen | zwischen 25 und 50 mL |
| Elutionspuffer | 20 mM Tris/Acetat + 0,1 mM $ZnCl_2$ pH 7,5 |
| Kontinuierlichen Gradient | 1000 mM bis 0 mM Ammoniumsulfat |
| Stufengradient | 20 mM Tris/Acetat + 0,1 mM $ZnCl_2$ pH 7,5 |
| Gradientenlänge | zwischen 4fache und 10fachen Säulenvolumen |
| Beladung (CPB/mL HIC-Gel) | zwischen 29,2 und 183 U/mL |

***Enzymaktivität***

Um die spezifische Aktivität der rekombinanten Carboxypeptidase B (npCPB) und der Carboxypeptidase B aus Schweinepankreas (*pig*CPB) zu bestimmen wird wie folgt vorgegangen. Zunächst wird die Volumenaktivität der CPB bestimmt. Als Substratlösung wird 0,015 M Hippuryl-Arginin (Fa. Sigma) in 0,05 M Tris/HCl-Puffer pH 7,8 gelöst. Weiterhin wird ein 50 mM Tris/HCl-Puffer pH 7,8 benötigt. Die Reaktionslösung besteht aus 0,5 mL Tris-Puffer, 0,1 mL der Substratlösung und 0,385 mL dest. Wasser. Die Reaktion wird mit 17μL CPB-Enzymlösung gestartet. Die photometrische Messung (ΔE) erfolgt 1 min lang in einer Quarzglasküvette mit einer Schichtdicke von 0,5 cm, bei einer Temperatur von 25°C und einer Wellenlänge von λ= 254 nm. Die CPB-Aktivität wird nach folgender Formel berechnet.

$$CPB[U/mL] = \frac{\Delta E * 1002 * Verdünnung}{0{,}349 * 0{,}5 * eingesetzteEnzymlösung}$$

Die zugehörige Proteinkonzentration der Enzymlösung wird photometrisch bei einer Wellenlänge von 280 nm in einer Quarzglasküvette mit einer Schichtdicke von 1cm und einer Temperatur zwischen 20 und 25°C bestimmt. Zunächst wird der Leerwert ermittelt indem nur die Absorption des Probenpuffers gemessen (E(Leerwert)). Der Probenpuffer besteht aus 0,033M Tris/HCl pH 8,0. Dann werden 0,05mL CPB-Lösung in 3 mL Probenpuffer verdünnt und ebenfalls die Absorption bestimmt (E(Probe)). Die Proteinkonzentration wird nach folgender Formel berechnet.

$$Proteingehalt[mg/mL] = \frac{10\,^{g}/_{L} * \Delta E(Probe)}{21{,}4} * 61$$

$$\Delta E(Probe) = E(Probe) - E(Leerwert)$$

| Enzym | Aktivität | Proteingeh. | Spez. Akt. |
|---|---|---|---|
| *np*CPB | 92,6 U/mL | 0,31 mg/mL | 298,7 U/mg |
| *pig*CPB (Archiv 28754, Fa. Merck) | 244,4U/mL | 0,94mg/mL | 260,0 U/mg |

## Patentansprüche

1. Nukleinsäure, codierend für Pro-Carboxypeptidase B (Pro-CPB) umfassend drei Abschnitte A, B und C, wobei Abschnitt A die Seq. ID Nr. 1, Abschnitt B die Seq. ID Nr. 2 und Abschnitt C die Seq. ID Nr. 3 aufweist.

2. Pro-Carboxypeptidase erhältlich durch Expression einer Nukleinsäure nach Anspruch 1.

3. Carboxypeptidase B, erhältlich durch Abspaltung der pro-Sequenz von Pro-CPB nach Anspruch 2 durch Trypsin.

4. Carboxypeptidase nach Anspruch 3 mit einer Enzymaktivität von mindestens 200 U/mg.

5. Expressionsvektor enthaltend eine Nukleinsäure gemäß Anspruch 1.

6. Transformierter nicht-menschlicher Organismus enthaltend einen Expressionsvektor gemäß Anspruch 5.

7. Verfahren zur Expression von Pro-CPB gemäß Anspruch 2, umfassend die Schritte:

   - Fermentieren eines transformierten Organismus gemäß Anspruch 6,
   - Induzieren der Expression,
   - Aufreinigen der Pro-CPB.

8. Verfahren zur Expression von Carboxypeptidase B gemäß Anspruch 3 oder 4 umfassend die Schritte:

   - Fermentieren eines transformierten Organismus gemäß Anspruch 6,
   - Induzieren der Expression,
   - Aktivierung durch Spaltung der Pro-CPB zu CPB,
   - Aufreinigung der CPB.

9. Pro-Carboxypeptidase mit der Sequenz gemäß Seq. ID Nr. 7.

10. Protein mit einer Aminosäuresequenz gemäß Seq. ID Nr. 8 mit mindestens 5 Mutationen ausgewählt aus der Gruppe D22H, S24N, E25I, R33T, A63T, E69K, C94V, E115Q, K120E, D135E, D137R, N138T, Q168P, D177E, Y184R, A186I, F191L, N194K, N240D, T245S, V246I, V250R, N254D, I295M, D309N, S314A, G318A, A319T, Y327H, S330K, S337A, N353D, F370Y, A381P, Q384E, V390I, N395S, T397V.

## Claims

1. A nucleic acid coding for pro-carboxypeptidase B (Pro-CPB), comprising three segments A, B and C, wherein segment A has the SEQ ID No. 1, segment B has the SEQ ID No. 2, and segment C has the SEQ ID No. 3.

2. A pro-carboxypeptidase obtainable by expression of a nucleic acid according to claim 1.

3. A carboxypeptidase B obtainable by cleaving the pro sequence from Pro-CPB according to claim 2 using trypsin.

**4.** The carboxypeptidase according to claim 3 having a specific enzymatic activity of at least 200 U/mg.

**5.** An expression vector containing a nucleic acid according to claim 1.

**6.** A transformed non-human organism containing an expression vector according to claim 5.

**7.** A process for expression of Pro-CPB according to claim 2, comprising the steps of:

- fermenting a transformed organism according to claim 6;
- inducing expression;
- purifying the Pro-CPB.

**8.** A process for expression of carboxypeptidase B according to claim 3 or 4, comprising the steps of:

- fermenting a transformed organism according to claim 6;
- inducing expression;
- activating by cleaving the Pro-CPB into CPB;
- purifying the CPB.

**9.** A pro-carboxypeptidase having the sequence according to SEQ ID No. 7.

**10.** A protein having an amino acid sequence according to SEQ ID No. 8 comprising at least 5 mutations selected from the group of D22H, S24N, E25I, R33T, A63T, E69K, C94V, E115Q, K120E, D135E, D137R, N138T, Q168P, D177E, Y184R, A186I, F191L, N194K, N240D, T245S, V246I, V250R, N254D, I295M, D309N, S314A, G318A, A319T, Y327H, S330K, S337A, N353D, F370Y, A381P, Q384E, V390I, N395S, T397V.

**Revendications**

**1.** Acide nucléique, codant pour la pro-carboxypeptidase B (pro-CPB) comprenant trois segments A, B et C, le segment A présentant la Seq ID N°1, le segment B la Seq ID N°2 et le segment C la Seq ID N°3.

**2.** Pro-carboxypeptidase pouvant être obtenue par l'expression d'un acide nucléique selon la revendication 1.

**3.** Carboxypeptidase B, pouvant être obtenue par le clivage de la séquence pro de pro-CPB selon la revendication 2 par la trypsine.

**4.** Carboxypeptidase selon la revendication 3 avec une activité enzymatique d'au moins 200 U/mg.

**5.** Vecteur d'expression contenant un acide nucléique selon la revendication 1.

**6.** Organisme non humain transformé contenant un vecteur d'expression selon la revendication 5.

**7.** Procédé pour l'expression de pro-CPB selon la revendication 2, comprenant les étapes suivantes :

- fermentation d'un organisme transformé selon la revendication 6,
- induction de l'expression,
- purification de la pro-CPB.

**8.** Procédé pour l'expression de la carboxypeptidase B selon la revendication 3 ou 4, comprenant les étapes suivantes :

- fermentation d'un organisme transformé selon la revendication 6,
- induction de l'expression,
- activation par la coupure de la pro-CPB en CPB
- purification de la CPB.

**9.** Pro-carboxypeptidase avec la séquence selon la Seq ID N°7.

**10.** Protéine avec une séquence d'acides aminés selon la Seq ID N°8 avec au moins 5 mutations sélectionnées dans le groupe D22H, S24N, E25I, R33T, A63T, E69K, C94V, E115Q, K120E, D135E, D137R, N138T, Q168P, D177E, Y184R, A186I, F191L, N194K, N240D, T245S, V246I, V250R, N254D, I295M, D309N, S314A, G318A, A319T, Y327H, S330K, S337A, N353D, F370Y, A381P, Q384E, V390I, N395S et T397V.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9623064 A
- US 5102789 A


### In der Beschreibung aufgeführte Nicht-Patentliteratur


- *Journal of Biological Chemistry,* 1999, vol. 174, 19925-19933